Europäisches Patentamt

(19) European Patent Office

Office européen des brevets

(11) Veröffentlichungsnummer: **0 129 235**
**B1**

(12) **EUROPÄISCHE PATENTSCHRIFT**

(45) Veröffentlichungstag der Patentschrift:
23.09.87

(21) Anmeldenummer: 84106941.2

(22) Anmeldetag: 18.06.84

(51) Int. Cl.⁴: **A 23 L 1/168,** A 23 L 1/216,
A 23 L 1/10, A 23 L 1/214,
A 23 K 1/14, A 23 K 1/18

(54) Verfahren zur Herstellung eines Produktes aus stärkehaltigen Ausgangsstoffen.

(30) Priorität: 21.06.83 CH 3375/83

(43) Veröffentlichungstag der Anmeldung:
27.12.84 Patentblatt 84/52

(45) Bekanntmachung des Hinweises auf die Patenterteilung:
23.09.87 Patentblatt 87/39

(84) Benannte Vertragsstaaten:
AT BE CH DE FR GB IT LI NL SE

(56) Entgegenhaltungen:
CH-A-348 037
DE-B-1 157 900
DE-C-952 764

(73) Patentinhaber: Gebrüder Bühler AG, CH- 9240 Uzwil
(CH)

(72) Erfinder: Ulmer, Karl, Waldeggstrasse 4, CH- 9500
Wil (CH)

Anmerkung: Innerhalb von neun Monaten nach der Bekanntmachung des Hinweises auf die Erteilung des europäischen Patents im
Europäischen Patentblatt kann jedermann beim Europäischen Patentamt gegen das erteilte europäische Patent Einspruch einlegen.
Der Einspruch ist schriftlich einzureichen und zu begründen. Er gilt erst als eingelegt, wenn die Einspruchsgebühr entrichtet worden
ist (Art. 99(1) Europäisches Patentübereinkommen).

LIBER, STOCKHOLM 1987

EP 0 129 235 B1

## Beschreibung

Bei der Herstellung von Kindermehl werden Komponenten aus Weizen, Reis usw. verwendet, die als Suspension im Wasser (Wassergehalt ca. 60 % des Gesamtgewichts), auf die Aussenfläche der Trommel eines Walzentrockners aufgegeben wird. Diese Oberfläche ist auf eine Temperatur von ca. 170° C ceheizt. Das bis auf weniger als 8 % Wassergehalt getrocknete Produkt wird als Folie von der Trommel abgestreift und einem Schlagwerk zugeführt, das die Folie zu Flocken zerkleinert. Das Produkt ist einwandfrei und es weist einen hohen Prozentsatz an gelatinisierter Stärke auf, was eine gute Verdaulichkeit ergibt.

Wegen der hohen Temperatur und vor allem wegen der grossen Menge von zu verdampfenden Wasser ist aber der Energiebedarf hoch. Deshalb ist man bei der vorliegenden Entwicklung auf die Idee gekommen, den Walzentrockner durch ein Walzwerk mit beheizten Walzen zu ersetzen, weil diese Maschine das Produkt verarbeiten kann, ohne dass es in Suspension gebracht werden muss. Die Menge des zu verdampfenden Wassers wird derart vermindert, dass die Bedürfnisse an Energie beträchtlich herabgesetzt werden.

Solche Walzwerke sind bekannt aber ihre Arbeitsbedingungen sind bisher für die Herstellung von Komponenten von Kindermehl mit hohem Gelatinisierungsgrad nicht geeignet gewesen. Das Schweizer Patent 348 037 betrifft z. B. die Herstellung von Nahrungsmittelflocken als Endprodukt. Die Ausgangsstoffe werden aber trocken in das Walzwerk gegeben, so dass ein Gelatinisieren nur beschränkt erfolgen kann.

Die Erfindung betrifft ein Verfahren zur Herstellung eines Produktes aus stärkehaltigen Ausgangsstoffen, wie Getreide, Hülsenfrüchten, Saaten, Kartoffeln, Maniok und dgl., wobei das Produkt beim Durchgang durch den Walzenspalt zwischen den geheizten Walzen eines Walzwerkes hindurchgeführt wird.

Die Aufgabe besteht in der Weiterbildung dieses Verfahrens, derart, dass durch seine Anwendung für vielfältige Ausgangsstoffe und Endprodukte, bei minimalem Eigenbedarf einen beliebigen Grad der Gelatinisierung erreicht werden kann, wobei sogar eine Retrogradation der Stärke möglich ist. Im Ausgangsstoff befindet sich die Stärke in Form von Stärkekörnern mit einer Hülle in welcher die Amylose und das Amylopektin, beide in Kristallform, enthalten sind. Beim Gelatinisieren werden nicht nur die Hülle sondern auch die Kristalle zerstört, was der Grund für die bessere Verdaulichkeit ist: sowohl die Hülle wie die Kristallform sind ein Hindernis für die Wirkung der Enzyme. Beim Weiterführen der thermischen Behandlung nach dem Gelatinisieren erfolgt das Retrogradieren der Stärke, wobei Kristalle wieder gebildet werden, aber nicht die Hülle, so dass ein mittlerer Widerstand gegenüber den Enzymen vorhanden ist.

Die Aufgabe wird durch die Kombination folgender Massnahmen gelöst:

a) der Feuchtigkeitsgehalt des Produktes vor dem Walzwerk beträgt 20-30 % des Gesamtgewichtes,

b) die Temperatur der Walzenoberfläche beträgt 100-200° C,

c) die Dicke des aus dem Walzenspalt zusammengepresst austretenden Produktes beträgt 0,05-0,2 mm.

Die angegebenen Bereiche erlauben es, den gewünschten Gelatinisierungsgrad zu erreichen, ohne dass überschüssige Feuchtigkeit verdampft werden muss, und ohne dass zu hohe Temperaturen notwendig sind, weil die relativ kleine Flockendicke einen guten Wärmeaustausch begünstigt.

In einer Weiterbildung des erfindungsgemässen Verfahrens wird das Produkt durch mehrere Walzenspalten hindurchgeführt. Durch die Verteilung der Behandlung zwischen mehreren Walzenspalten ist es möglich, mit einer tieferen Temperatur zu arbeiten als bei einem einzigen Walzenspalt, was zur Schonung von temperaturempfindlichen Produkten beiträgt. Die Temperaturen der Walzenoberfläche können in einem Bereich von 100-150° C gewählt werden, während dem der Feuchtigkeitsgehalt des Produktes vor dem Walzwerk meistens 25-30 % beträgt.

Wenn das Produkt vor dem Walzenspalt extrudiert wird, ist es möglich, dem ersten Walzenspalt dieses Produkt in einer schon pastösen, vorhomogenisierten Form zuzuführen. Damit wird es möglich, mit dem Walzwerk eine fertige Mischung zu verarbeiten, dessen einzelne Komponenten sonst in korniger pulvriger oder mehliger Form durch das Walzwerk einzeln verarbeitet werden sollten, weil grosse Unterschiede ihre physikalischen Eigenschaften zu Schwierigkeiten führen würden. Wegen der Behandlungstemperatur verlässt eine solche Mischung das Walzwerk in einwandfreien bakteriologischem Zustand, der leichter beibehalten werden kann, wenn ein nachträgliches Mischen entfällt.

Bei der Verwendung eines einzigen Walzenpaares ist es für viele Produkte vorteilhaft die Walzen mit einem Geschwindigkeitsverhältnis von 1,05:1 bis 1,25:1 anzutreiben.

Wenn das Produkt durch mehrere Walzenspalten hindurchgeführt wird, wobei die Zwischenwalzen an die Bildung von zwei Walzenspalten teilnehmen, muss im allgemeinen das Geschwindigkeitsverhältnis der Walzen eines einzelnen Walzenspaltes höher sein, und beträgt mit Vorteil 2:1 bis 3:1, damit das durch den Walzenspalt hindurchgepresste, teigig gewordene, Produkt mit Sicherheit durch die schneller drehenden Walze mitgenommen wird. Damit steigt auch die Geschwindigkeit von der ersten bis zur letzten Walze. Deshalb kann man mit Vorteil Walzwerke benützen, deren Konstruktion weitgehend gleich ist, wie diejenige der Dreiwalzwerke und Fünfwalzwerke der Farben- und Schokoladenindustrie. Der Unterschied besteht darin, dass die Walzen nicht gekühlt, sondern stark geheizt werden.

Wenn das Produkt auf der schnellen Walze eines Walzwerkes durch eine Flockenbrechwalze behandelt wird, ergeben sich Vorteile in der Disposition einer Anlage, weil eine getrennte Maschine entfällt.

Die Anwendungen des Verfahrens nach der Erfindung sind ausserordentlich vielfältig. Es kann z. B. bei der Herstellung von Kindermehl, von Instant-Produkten, von Kartoffelflocken, von Petfood (unter Petfood werden Futter für Haustiere verstanden) und andere mehr verwendet werden.

Es zeigen:
Fig. 1 das Schema einer ersten Ausführungsform der Erfindung;
Fig. 2 das Schema einer zweiten Ausführungsform der Erfindung; und
Fig. 3 das Schema einer dritten Ausfuhrungsform der Erfindung.

In den Figuren sind die einzelnen Maschinen symbolisch dargestellt. Bei den Walzwerken z. B. ist die Anzahl Walzen gezeigt, aber nicht im angepressten Zustand.

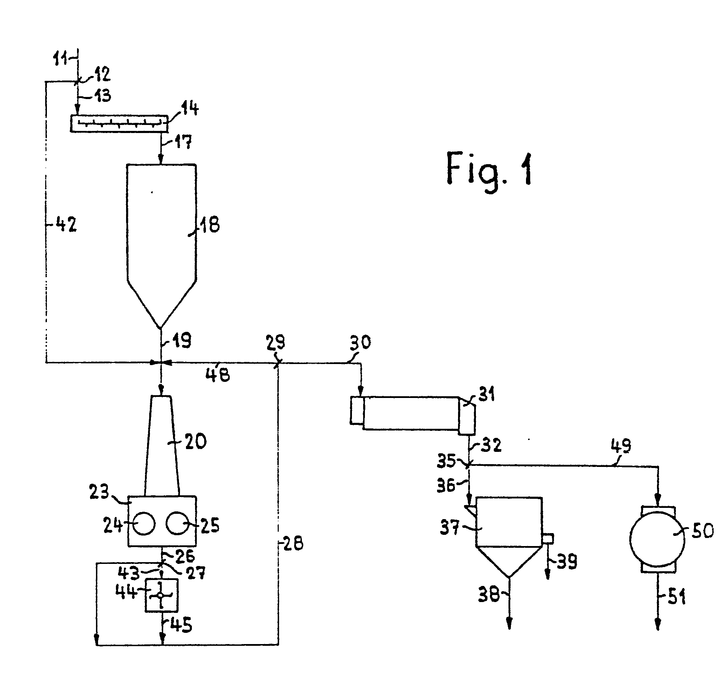

Die Ausgangsstoffe kommen durch eine Zufuhrleitung 11 (Fig.1) über eine Rohrweiche 12 und eine Speiseleitung 13 in einen Intensivnetzapparat 14. Darin dreht mit grosser Geschwindigkeit eine nicht dargestellte Trommel, deren Umfang mit einer grossen Anzahl Schaufeln bestückt ist. In der Nähe des Eingangs wird den Ausgangsstoffen Wasser zugesetzt. Die Schaufeln fördern das Produkt kontinuierlich entlang der Trommel und begünstigen das Eindringen des Wassers in die Körner. Das Produkt verlässt den Intensivnetzapparat 14 über ein Fallrohr 17 und gelangt in einen Abstehkasten 18, wo es 4 bis 6 Stunden verbleibt, bis das Wasser in den Körnern gleichmässig verteilt ist. Beim Verlassen des Abstehkastens 18 beträgt dieser Wassergehalt meistens 17 bis 20 % des Gesamtgewichts. Über eine Austragsleitung 19 gelangt dann das Produkt in einen Dämpfer 20, in welchem er 20 bis 30 Minuten verbleibt. Im Dämpfer 20 wird das Produkt durch Zuführen von Dampf konditioniert. Es erreicht eine Temperatur von z. B. 100° C und der Feuchtigkeitsgehalt erreicht 20 bis 30 % des Gesamtgewichts. Vom Dämpfer 20 wird das Produkt durch die nicht dargestellte Speisevorrichtung eines Walzwerkes 23 kontinuierlich ausgetragen und den gegenläufig drehenden Walzen 24, 25 zugeführt. Die Walzen sind geheizt, damit ihre Oberfläche eine Temperatur von 100 bis 200° C erreicht. Sie sind miteinander durch Keilriemen verbunden und ihr Geschwindigkeitsverhältnis beträgt von 1,05:1 bis 1,25:1. (In der Fachsprache des Müllers heisst es, dass das Differenzial 5 % bis 25 % beträgt). Bei der gewählten Ausführungsform des Walzwerks 23 beträgt der Durchmesser der Walzen 600 mm und sie werden gegeneinander mit einer Kraft angepresst, die 300 bis 450 kg/cm Walzenlänge beträgt.

Das aus dem Walzenspalt zusammengepresst austretende Produkt bildet eine Folie von einer Dicke von 0,1 bis 0,2 mm. In vielen Fällen verfällt diese Folie schon innerhalb des Walzwerkes in kleinere Stücke, die über ein Fallrohr 26, eine Rohrweiche 27, eine Förderleitung 28, eine Rohrweiche 29 und eine Förderleitung 30 einem Wirbelschichtapparat 31 zugeführt werden. Dort verbleiben die Produktstücke 3 bis 6 Minuten und werden gekühlt und falls notwendig bis auf einen Wassergehalt von weniger als 5 % des Gesamtgewichts getrocknet. Sie verlassen den Wirbelschichtapparat 31 über eine Austragleitung 32, eine Rohrweiche 36, die sie einem Schleudersieb 37 zuführt. Dieses Schleudersieb 37 besteht aus einer nicht dargestellten festen Siebtrommel in welcher ein Schleuderwerk dreht, das die Stücke gegen die Innenfläche der Siebtrommel wirft. Das Schleudersieb 37 arbeitet sowohl als Siebmaschine, wie auch als Zerkleinerungsmaschine. Die zu Flocken zerkleinerten Produktstücke treten durch die Maschen der Siebtrommel hindurch und werden über die Austragsleitung 38 entladen. Sollten gegen Erwarten zu wenig zerkleinerte Produktstücke die Siebtrommel als Abstösse verlassen, so können sie über eine Austragsleitung 39 ausgetragen werden. Sie können z. B. in die Speiseleitung 36 zurückgeführt werden.

Von der Rohrweiche 12 geht eine Umgehungsleitung 42 bis zur Austragsleitung 19. Sie erlaubt es, den Intensivnetzapparat 14 zu vermeiden, und die Ausgangsstoffe direkt in den Dämpfer 20 einzuspeisen. Dies ist z. B. der Fall für feine Ausgangsstoffe.

Falls die aus dem Walzenspalt des Walzwerkes 23 verlassende Folie nicht von selbst in kleinere Stücke zerfällt, so wird sie über die Rohrweiche 27 und eine Zuleitung 43 einem Haspelgerät 44 zugeführt, die sie zerkleinert. Die zerkleinerten Stücke werden über eine Förderleitung 45 zur Förderleitung 28 zugeführt.

Beim Anfahren der Anlage kann es geschehen, dass die aus dem Walzwerk 24 kommenden Produktstücke noch zu trocken sind. Die Förderleitung 26 wird dann über die Rohrweiche 29 mit der Rückfuhrleitung 48 verbunden, die die Produktstücke über die Austragsleitung 19 wieder zum Dämpfer 20 bringt. Falls die Anlage nicht Flocken, sondern Mehl herstellen muss, verbindet dann die Rohrweiche 35 die Austragsleitung 32 mit einer Förderleitung 49, die die aus den Wirbelschichtapparat ausgetragenen Produktstücke einer Vermahlungsmaschine 50, z. B. einer Schlagmühle, zugeführt, die diese Produktstücke zu Mehl zerkleinert. Das Mehl wird über eine Austragsleitung 51 der Anlage entnommen.

Es werden nun zwei Verarbeitungsbeispiele für die Anlage der Fig. 1 gegeben.

**Beispiel 1**

- es wird eine Komponente für Kindermehl hergestellt;
- der Gelatinisierungsgrad soll 50 % betragen, d.h. 50 % der im Ausgangsstoff enthaltenen Stärke muss gelatinisiert werden;
- Ausgangsstoff ist Reisbruch (ungefähr zwei Stück per Reiskorn);
- der Reisbruch geht über die Umgehungsleitung 42 direkt in den Dämpfer 22,
- Konditionierung auf 20 % Wassergehalt im Verhältnis zum Gesamtgewicht;
- Konditionierzeit 20 Minuten;
- Temperatur der Walzenoberfläche 120°C;
- Anpresskraft: 300 kg/cm Walzenlänge;
- Geschwindigkeit der Walzenoberfläche: ca. 5 m/sec;
- Geschwindigkeitsverhältnis der Walzen: 1,05:1;
- Flockendicke: 0,2 mm;
- Flockendurchmesser bis 10 mm, z. B. 2 mm.

**Beispiel 2**

- Herstellung einer Komponente für die Herstellung von Kindermehl;
- der Gelatinisationsgrad soll 85 % (mit β-Amylase gemessen) betragen;
- Ausgangsstoff: Weizen geschält;
- Netzen im Intensivnetzapparat 14 auf einen Wassergehalt von 20 % vom Gesamtgewicht;
- Abstehzeit im Abstehkasten 18: 4 Stunden;
- Konditionierzeit im Dämpfer 20: 25 Minuten;
- Feuchtigkeitsgehalt am Ausgang des Dämpfers 20: 27 %;
- Walzentemperatur 190°C;
- Anpresskraft: 300 kg/cm Walzenlänge;
- Walzengeschwindigkeit: 5 m/sec;
- Differential: 20 %;
- Flockendicke: 0,1 mm;
- Flockendurchmesser: 3 mm.

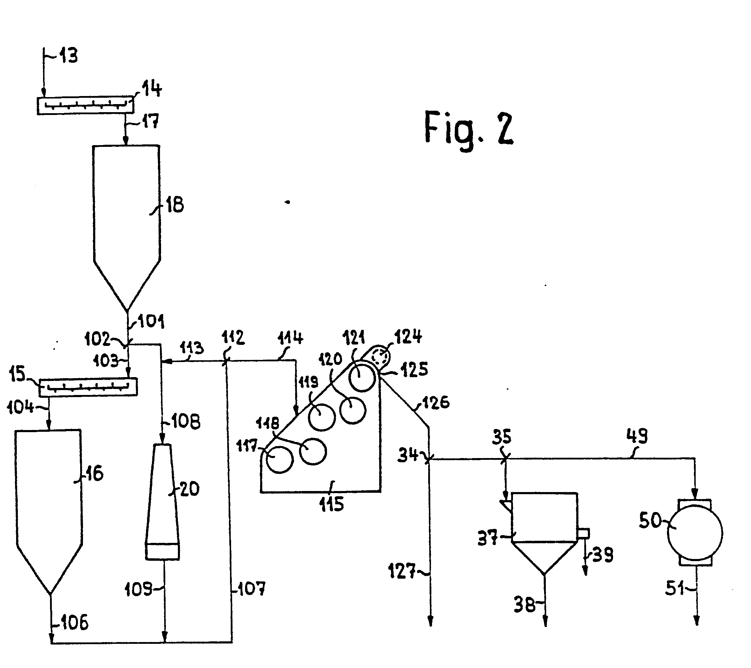

Bei der Anlage nach Fig. 2 erhalten Apparate, die gleich sind wie in Fig. 1, das gleiche Bezugszeichen. Ein zweiter Intensivnetzapparat erhält das Bezugszeichen 15 und ein zweiter Abstehkasten das Bezugszeichen 16.

Das Ausgangsprodukt wird aus dem Abstehkasten 18 über die Austragsleitung 101, die Rohrweiche 102 und die Speiseleitung 103 dem zweiten Intensivnetzapparat 15 zugeführt, und verlässt es über das Fallrohr 104 um in den zweiten Abstehkasten 16 zu gelangen, wo es ebenfalls 4 bis 6 Stunden verbleibt. Das Produkt wird kontinuierlich aus dem zweiten Abstehkasten 16 über die Austragsleitung 106 entladen und gelangt über eine Förderleitung 107, eine Rohrweiche 112 und eine Zuführleitung 114 zum Fünfwalzwerk 115. Durch Umstellung der Rohrweiche 102 kann das Produkt aus dem ersten Abstehkasten 18 über die Speiseleitung 108 in den Dämpfer 20 gelangen, aus welchem es kontinuierlich über die Austragsleitung 109 in die Förderleitung 107 gelangt. Ist das Produkt in der Förderleitung 107 noch zu trocken, kann es durch Umstellen der Rohrweiche 112 über die Rückführleitung 113 dem Dämpfer 20 wieder zugeführt werden.

Jede Walze 117 bis 121 des Fünfwalzwerkes 115 bildet mit den Nachbarwalzen einen Walzenspalt. Jede Walze dreht gegenläufig zur Nachbarwalze, mit welcher sie über Zahnräder verbunden ist. Die Drehzahlen der Walzen steigen von Walze zu Walze der Reihe nach. Dies ist die Bedingung dafür, dass das Produkt von einer auf die nächstfolgende Walze übernommen wird. Praktisch handelt es sich um das Walzwerk, das in der Schokoladenindustrie zur Verfeinerung der Schokoladenmasse vor dem Conchieren und dem Giessen verwendet wird. Ein Unterschied liegt darin, dass die Walzen nicht gekühlt werden wie in der Schokoladenindustrie, sondern geheizt werden. Das Geschwindigkeitsverhältnis zwischen den Walzen eines Walzenpaares beträgt von 2:1 bis 3:1. Der Walzendurchmesser beträgt 400 mm, die Geschwindigkeit der Oberfläche der ersten Walze wird zwischen 0,6 und 1 m pro Sekunde gewählt. Die Walzen werden gegeneinander mit einer Kraft von 120 bis 130 kg/cm Walzenlänge angepresst. Die Temperatur der Oberfläche der Walzen beträgt von 100 bis 150°C. Die Dicke des aus dem letzten Walzenspalt (zwischen den Walzen 120, 121) zusammengepressten austretenden Produkte beträgt 0,05 bis 0,1 mm. Dem Fünfwalzwerk 115 wird ein Produkt zugeführt, dessen Feuchtigkeitsgehalt bei Getreide 25 bis 30 % des Gesamtgewichts beträgt.

Der schnellen Walze 121 ist eine Flockenbrechwalze 124 zugeordnet, die die Produktfolie auf der Walze 121 in Flocken zerlegt. Das Produkt wird von der Walze 121 durch den Abstreifer 125 entnommen und über die Förderleitung 126 und die Rohrweiche 34 bis zur Rohrweiche 35 gebracht, und von dort, wie bei der Anlage der Fig. 1 entweder dem Schleudersieb 37 oder der Vermahlungsmaschine 50. Zusätzlich kann die Rohrweiche 35 das Produkt einer Austragsleitung 127 zuleiten, falls die Flockenbrechwalze 124 schon Flocken mit den gewünschten Dimensionen erzeugt hat.

Es werden nun zwei Arbeitsbeispiele für die Anlage nach Fig. 2 gegeben.

**Beispiel 3**

- Herstellung einer Komponente für Kindermehl;
- Ausgangsstoff: Gerstengrütze (3 bis 4 Stücke pro Gerstenkorn);
- erwünscht ist ein Gelatisierungsgrad von 50 %;
- Vornetzen im Intensivnetzapparat 14 auf 20 % Wassergehalt der Gesamtgewichts;
- Abstehzeit: 4 Stunden im Abstehkasten 18;
- Konditionieren im Dämpfer 20 auf 26 % Wassergehalt
- Konditionierungszeit: 25 Minuten;
- Walzentemperatur: 100°C.;
- Anpresskraft 120 kg/cm Walzenlänge
- Geschwindigkeitsverhältnis der schnellen Walze zur langsamen Walze eines Walzenpaares: 2:1;
- Oberflächengeschwindigkeit der ersten Walze: 0,6 m/S;
- die Flockendicke beträgt 0,05 bis 0,1 mm.

**Beispiel 4:**

- Herstellung eines Trägerstoffes für pharmazeutische Präparate;
- Ausgangsprodukt: Weizen, ganze geschälte Körner;
- Gewünscht wird eine totale Gelatinisierung mit starker Retrogradation;
- Vornetzen im Intensivnetzapparat 14 auf 20 % Wassergehalt relativ zum Gesamtgewicht;
- Abstehzeit im Abstehkasten 18: 4 Stunden
- Nachnetzen auf dem zweiten Intensivnetzapparat 15 auf 28 % Wassergehalt;
- Abstehzeit im Abstehkasten 16: 4 Stunden;
- Oberflächentemperatur der Walzen 117, 118: 100°C.;
- Oberflächentemperatur der Walzen 119 bis 121: 150°C;
- Geschwindigkeitsverhältnis zwischen den Walzen eines Walzenpaares 2,5:1
- Anpresskraft zwischen den Walzen: 130 kg/cm Walzenlänge;
- Geschwindigkeit der ersten Walze: 0,6 m/S
- die Flockendicke beträgt 0,05 mm.

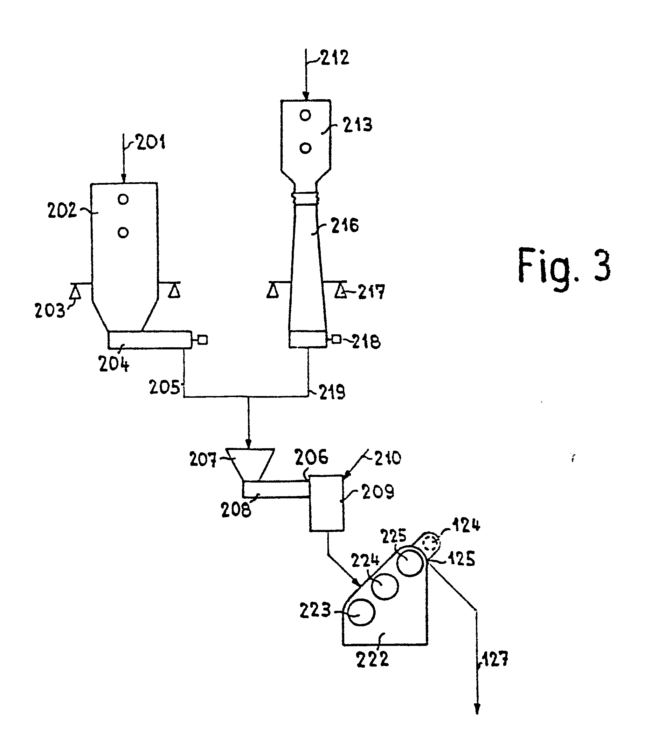

Bei der Anlage nach Fig. 3 führt eine Zufuhrleitung 201 Mehlkomponenten aus einer nicht dargestellten Vormischerei in einen Behälter 202, der auf Gewichtsmessdosen 203 angeordnet ist. Die vorgemischten Mehlkomponenten werden durch eine Austragsschnecke 204 dosiert vom Behälter 203 über ein Fallrohr 205 in den Speisetrichter 207 eines Misch-Extruders 206 gefördert. Dieser weist eine Speiseschnecke 208 auf, die auch als Mischstufe dient und das Produkt einem vertikalen Extruder 209 zuführt. Beim Übergang zwischen Speiseschnecke 208 und Extruder 209 besteht ein freier Raum, in welchem eine Einrichtung 210 für die Zugabe von Feuchtigkeit auf das von der Speiseschnecke 208 in die nicht dargestellte Schnecke des Extruders 209 fallende Produkt. Es können Wasser, Dampf oder andere flüssige und dampfförmige Komponente zugegeben werden. Für den Mischer-Extruder 206 kann mit Vorteil die Konstruktion nach der Europäischen Patentanmeldung mit Veröffentlichungsnummer EP-A 0 036 983, Anmeldenummer 81 101 886.0, der Anmelderin verwendet werden. Bei der Patentanmeldung EP-A 0 036 985 hat die vertikale Extruderschnecke nur wenige Windungen und sie treibt Versalbungswerkzeuge an, die vor der Lochplatte angeordnet sind, durch welche hindurch das Produkt extrudiert wird. Besonders günstig ist eine Anordnung mit zwei hintereinander angeordneten Versalbungswerkzeugen, deren radial liegenden Arme gegeneinander versetzt sind.

Eine weitere Zuführleitung 212 führt körnige Komponenten, einzeln oder als Mischung, von einem nicht dargestellten Intensivnetzapparat zu einem Abstehkasten 213, der einen Dämpfer 216 speist, der auf Gewichtsmessdosen 217 ruht. Eine Austragswalze 218 führt das konditionierte Produkt über ein Fallrohr 219 zum Speisetrichter des Mischer-Extruders 206. Es wäre denkbar, statt des Dämpfers 216 oder zusätzlich zu diesem, eine zweite Netzerei mit Intensivnetzapparat und Abstehkasten vorzusehen.

Vom Extruder 209 fällt das teigige Produkt direkt in ein Dreiwalzwerk 222 und wird durch den Walzenspalt zwischen den beheizten Walzen 223, 224 und dann durch den Walzenspalt zwischen den Walzen 224, 225 hindurchgepresst. Wie beim Fünfwalzwerk 115 der Fig. 2 erhöht sich die Geschwindigkeit von einer Walze zur anderen. Die Kopplung der Walzen 223-225 durch Zahnräder ist analog wie beim Fünfwalzwerk 115 der Fig. 2. Beim Dreiwalzwerk 222 handelt es sich um ein Walzwerk wie es in der Farbenindustrie, z. B. für die Verarbeitung von zähen Druckfarben, verwendet wird. Auch hier liegt der Unterschied darin, dass die Walzen 223-225 geheizt anstatt gekühlt sind. Wie beim Fünfwalzwerk 115 ist die letzte, schnelle Walze 225, mit einer Flockenbrecherwalze 124 und einem Abstreifer 125 versehen, und die Weiterverarbeitung kann genau wie bei der Fig. 2 vorgesehen, d.h. wahlweise über ein Schleudersieb 37, eine Schlagmühle 50 oder eine direkte Austragsleitung 127.

Weil in der Anlage nach Fig. 3 eine ganze Mischung verarbeitet wird, kann nach dem Walzwerk direkt abgepackt werden, was bakteriologisch sehr günstig ist. Vorteilhaft ist auch die Feuchtigkeitszugabe 210, die eine Korrektur des Feuchtigkeitsgehaltes direkt vor der Flockierung noch erlaubt. Es wäre möglich, auch die Speiseschnecke 208 und den Extruder 209 zu heizen.

Es wird ein Arbeitsbeispiel für die Anlage nach Fig. 3 gegeben.

**Beispiel 5**

- Herstellung einer Getreidemischung für Kindernährmittel;
- Ausgangsstoffe: 50 % Reisbruch, 30 % Weizen geschält, 10 % Hafergrütze, 10 % Gerstengrütze, vorgenetzt auf 20 % Wassergehalt;
- erwünscht ist ein Gelatinisierungsgrad von 80 %;
- Konditionieren im Dämpfer 216 auf 27 % Wassergehalt;

- Konditionierungszeit: 30 Minuten;
- Extrudieren auf Mischer-Extruder 206;
- Walzentemperatur: 140°C;
- Differential eines Walzenpaares: 2,5:1
- Geschwindigkeit der ersten Walze: 0,9 m/S;
- Anpresskraft: 130 kg/cm;
- die Flockendicke beträgt 0,05 mm.

**Patentansprüche**

1. Verfahren zur Herstellung eines Produktes aus stärkehaltigen Ausgangsstoffen, wie Getreide, Hülsenfrüchten,Saaten, Kartoffeln, Maniok und dgl., wobei das Produkt beim Durchgang durch den Walzenspalt zwischen den geheizten Walzen eines Walzwerkes hindurchgeführt wird, gekennzeichnet durch die Kombination folgender Massnahmen
    a) der Feuchtigkeitsgehalt des Produktes vor dem Walzwerk beträgt 20-30 % des Gesamtgewichts
    b) die Temperatur der Walzenoberfläche beträgt 100-200°C
    c) die Dicke des aus dem Walzenspalt zusammengepresst austretenden Produktes beträgt 0,05-0,2 mm.

2. Verfahren nach Anspruch 1, dadurch gekennzeichnet, dass das Produkt durch mehrere Walzenspalte hindurchgeführt wird.

3. Verfahren nach einem der Ansprüche 1, 2, dadurch gekennzeichnet, dass das Produkt vor dem Walzenspalt extrudiert wird.

4. Verfahren nach einem der Ansprüche 1 bis 3, dadurch gekennzeichnet, dass die Walzen eines Walzenspaltes mit einem Geschwindigkeits-Verhältnis von 1,05:1 bis 1,25:1 angetrieben werden.

5. Verfahren nach einem der Ansprüche 2, 3, dadurch gekennzeichnet, dass bei den einzelnen Walzenspalten die Walzen mit einem Geschwindigkeitsverhältnis von 2:1 bis 3:1 angetrieben werden.

6. Verfahren nach einem der Ansprüche 1 bis 5, dadurch gekennzeichnet, dass das Produkt auf der schnellen Walze eines Walzenpaares durch eine Flockenbrechwalze behandelt wird bevor es abgenommen wird.

7. Verfahren nach einem der Ansprüche 2, 3, 5, 6, dadurch gekennzeichnet, dass die Temperatur der Walzenoberfläche 100-150°C beträgt bei totaler Gelatinisation.

8. Verfahren nach Anspruch 7, dadurch gekennzeichnet, dass der Feuchtigkeitsgehalt des Ausgangsproduktes 25-30 % des Gesamtgewichtes beträgt.

9. Anwendung des Verfahrens nach einem der Ansprüche 1 bis 8 zum Herstellen von Komponenten für Kindermehl.

10. Anwendung des Verfahrens nach einem der Ansprüche 1 bis 8 zum Herstellen eines Instant Produktes.

11. Anwendung des Verfahrens nach einem der Ansprüche 1 bis 8 zum Herstellen von Petfood.

**Claims**

1. Process for producing a product from starch-containing raw materials, such as grain, legumes, seeds, potatoes, manioc and the like, wherein the product as it passes through the roll gap is passed between the heated rolls of a roll mill, characterised by the combination of the following measures
    a) the moisture content of the product upstream of the roll mill amounts to 20-30 % of the total weight,
    b) the temperature of the roll surface amounts to 100-200°C
    c) the thickness of the product issuing in compressed form from the roll gap amounts to 0.05-0.2 mm.

2. Process according to claim 1, characterised in that the product is passed through a plurality of roll gaps.

3. Process according to one of claims 1 and 2, characterised in that the product is extruded upstream of the roll gap.

4. Process according to one of claims 1 to 3, characterised in that the rolls of a roll gap are driven at a speed ratio of 1.05:1 to 1.25:1.

5. Process according to one of claims 2 and 3, characterised in that at the individual roll gaps the rolls are driven with a speed ratio of 2:1 to 3:1.

6. Process according to one of claims 1 to 5, characterised in that the product is treated by a flake-producing breaking roll when on the fast roll of a pair of rolls before it is taken away.

7. Process according to one of claims 2, 3, 5, 6, characterised in that the temperature of the roll surface amounts to 100-150°C, with total gelatinisation.

8. Process according to claim 7, characterised in that the moisture content of the raw product amounts to 25-30 % of the total weight.

9. Use of the process according to one of claims 1 to 8 for the production of constituents for powdered infant food.

10. Use of the process according to one of claims 1 to 8 for the production of an instant product.

11. Use of the process according to one of claims 1 to 8 for the production of pet food.

**Revendications**

1. Procédé de fabrication d'un produit à partir de matières de départ contenant des amidons, telles que les céréales, les plantes légumineuses, les graines, les pommes de terre, le manioc et d'autres matières du même genre, procédé selon lequel le produit, lors de son passage par la passe des cylindres, est amené à passer entre les cylindres chauffés à'un appareil lamineur, ce procédé étant caractérisé par la combinaison des mesures qui sont indiquées ci-après:
    a) la teneur en humidité du produit, avant le passage de celui-ci dans l'appareil lamineur, est de 20 à 30 % du poids total;

b) la température de la surface des cylindres est de 100 à 200°C, et

c) l'épaisseur du produit sortant à l'état comprimé de la passe des cylindres est de 0,05 à 0,2 mm.

2. Procédé suivant la revendications 1, caractérisé en ce que le produit est amené à passer par plusieurs passes de cylindres.

3. Procédé suivant l'une ou l'autre des revendications 1 et 2, caractérisé en ce que le produit est extrudé avant son passage par la passe des cylindres.

4. Procédé suivant l'une quelconque des revendications 1 à 3, caractérisé en ce que les cylindres formant une passe sont commandés à un rapport de vitesses de 1,05/1 à 1,25/1.

5. Procédé suivant l'une ou l'autre des revendications 2 et 3, caractérisé en ce que pour les différentes passes, les cylindres sont commandés à un rapport de vitesses de 2/1 à 3/1.

6. Procédé suivant l'une quelconque des revendications 1 à 5, caractérisé en ce que le produit se trouvant sur le cylindre rapide d'une passe de cylindres est traité par un cylindre de division en flocons avant d'être enlevé de ce cylindre rapide.

7. Procédé suivant l'une quelconque des revendications 2, 3, 5 et 6, caractérisé en ce que la température de la surface des cylindres est de 100 à 150°C pour une gélatinisation totale.

8. Procédé suivant la revendication 7, caractérisé en ce que la teneur en humidité du produit de départ est de 25 à 30 % du poids total.

9. Application du procédé suivant l'une quelconque des revendications 1 à 8 à la préparation de composants de farine pour enfants.

10. Application du procédé suivant l'une quelconque des revendications 1 à 8 à la fabrication d'un produit à préparation instantanée.

11. Application du procédé suivant l'une quelconque des revendications 1 à 8 à la fabrication de "petfood".

Fig. 1

Fig. 2

Fig. 3